# EUROPÄISCHE PATENTANMELDUNG

(11) **EP 3 786 977 A1**
(43) Veröffentlichungstag der Anmeldung: **03.03.2021**
(21) Anmeldenummer: 19194299.4
(22) Anmeldetag: 29.08.2019
(51) Int. Cl.: G16H 50/20

(54) **COMPUTERIMPLEMENTIERTES VERFAHREN UND ELEKTRONISCHES SYSTEM ZUR PROGNOSE EINES ENTBINDUNGSZEITPUNKTS**

(71) Anmelder: WPmed GbR, 80331 München (DE)
(72) Erfinder: Pressmar, Oliver, 80331 München (DE); Weissenbacher, Ernst-Rainer, 80331 München (DE)
(74) Vertreter: Grünecker Patent- und Rechtsanwälte PartG mbB

(57) **Zusammenfassung**

Die Erfindung betrifft ein computerimplementiertes Verfahren zur Prognose des Entbindungszeitpunkts einer schwangeren Frau, mit den Schritten: Erfassen von wenigstens einem Körperparameter der schwangeren Frau 1 zu mehreren Zeitpunkten, Durchführen einer Mustererkennung bezüglich des zeitlichen Verlaufs des wenigstens einen Körperparameters durch eine Berechnungseinheit, und Berechnen eines prognostizierten Entbindungszeitpunkts oder -zeitraums auf Basis der Mustererkennung. Die Erfindung betrifft weiterhin ein elektronisches System zur Prognose des Entbindungszeitpunkts einer schwangeren Frau.

## Beschreibung

Die vorliegende Erfindung betrifft ein computerimplementiertes Verfahren und ein elektronisches System zur Prognose eines Entbindungszeitpunkts einer schwangeren Frau, sowie ein diesbezügliches Client-Server-System.

Im Stand der Technik ist es bekannt, den monatlichen Zyklus bzw. den Fruchtbarkeitszeitraum einer Frau mittels elektronischer Hilfsmittel zu berechnen. So wird in der WO 2016/131630 A1 ein Armband offenbart, mit dem die Hauttemperatur, der Hautwiderstand und der Puls bestimmt werden können, um dadurch einen Fruchtbarkeitszeitraum berechnen. Dabei wird ein selbstlernender Algorithmus eingesetzt, der sich an individuelle Benutzerinnen anpassen kann. Weiterhin ist es beispielsweise bekannt, die Körpertemperatur einer Frau möglichst durchgehend zu erfassen, um den Fruchtbarkeitszeitraum zu bestimmen. Dies kann beispielsweise durch einen Sensor in der Vagina erfolgen. Weiterhin ist es aus der US 2007/0015285 A1 bekannt, einen Hormonwert zu bestimmen, um den Fruchtbarkeitszeitraum einer Frau zu ermitteln. Der vorgenannte selbstlernende Algorithmus passt sich dabei an die jeweilige individuelle Benutzerin an, was durch den sich wiederholenden Verlauf des Monatszyklus ermöglicht wird.

Es ist bekannt, ein theoretisches Entbindungsdatum ausgehend vom Datum der letzten Regelblutung mittels der Naegele-Regel vorherzubestimmen. So können beispielsweise Vorrichtungen, die ausgehend von Temperaturmessungen den Eisprung vorhersagen, am Beginn einer Schwangerschaft eine Angabe für das theoretische Entbindungsdatum machen, wie beispielsweise in der JP 2009-045155 und JP 2007-068839 offenbart. Allerdings ist die Berechnung des Entbindungsdatums eher ungenau, und erfolgt ohne Berücksichtigung des tatsächlichen Schwangerschaftsverlaufs.

In der EP 0 648 335 B1 wird offenbart, dass bei Schwangerschaften mit einem Risiko einer Frühgeburt oder bei übertragenen Schwangerschaften mittels eines Testkits die Präsenz eines Hormons in der Vagina der schwangeren Frau ermittelt werden kann, wodurch bestimmt werden kann, ob die Entbindung unmittelbar bevorsteht. Weiterhin wurde in medizinischen Studien ermittelt, dass es bei schwangeren Frauen in den Tagen vor der Entbindung zu einer Veränderung der Körpertemperatur kommt. Die diesbezügliche Erfassung und Auswertung ist für schwangere Frauen aber relativ kompliziert, und zudem durch andere Faktoren überlagert, sodass eine Prognose des Entbindungszeitpunktes ohne ärztliche Unterstützung nicht zuverlässig möglich ist. Die vorliegende Erfindung hat die Aufgabe, einer schwangeren Frau ein Verfahren und ein elektronisches System an die Hand zu geben, mit denen die Prognose eines Entbindungszeitpunktes sicher und in einfacherer Form ermöglicht wird.

Die Erfindung stellt ein computerimplementiertes Verfahren zur Prognose des Entbindungszeitpunktes einer schwangeren Frau bereit, bei dem wenigstens ein Körperparameter einer schwangeren Frau zu mehreren Zeitpunkten erfasst wird, eine Mustererkennung bezüglich des zeitlichen Verlaufs des wenigstens einen Körperparameter durchgeführt wird, und ein prognostizierter Entbindungszeitpunkt oder -zeitraum auf Basis der Mustererkennung berechnet wird.

Insbesondere wird der Entbindungszeitpunkt oder -zeitraum visuell, akustisch oder haptisch wiedergegeben, bevorzugt visuell angezeigt. Zusätzlich zur Anzeige des Entbindungszeitpunktes kann eine mögliche Zeitabweichung angezeigt werden, die die Berechnungsgenauigkeit der Prognose wiedergibt. Alternativ kann ein prognostizierter Entbindungszeitraum angegeben werden, dessen Länge die Berechnungsdauer wiedergibt. So kann insbesondere eine Wahrscheinlichkeit angezeigt oder ausgewählt werden, gemäß der erwartet werden kann, dass die Entbindung innerhalb der vorgenannten Zeitabweichung oder innerhalb der vorgenannten Entbindungszeitraums stattfinden wird. So kann mit zunehmender Körperparametererfassung insbesondere eine immer höhere Wahrscheinlichkeit hinsichtlich der Prognose bei gleichbleibender Zeitabweichung oder gleichbleibendem prognostiziertem Entbindungszeitraum angezeigt werden, oder eine immer kleiner werdende Zeitabweichung oder ein immer kleiner werdender prognostizierter Entbindungszeitraum bei gleichbleibender Wahrscheinlichkeit hinsichtlich der Prognose. Bei einem prognostizierten Entbindungszeitpunkt handelt es sich aufgrund der Prognoseunsicherheit insbesondere um den statistischen Mittelpunkt eines prognostizierten Entbindungszeitraums.

Im Gegensatz zum Monatszyklus einer Frau handelt es sich beim Verlauf einer Schwangerschaft nicht um einen zyklischen Ablauf, an den ein selbstlernender Algorithmus angepasst werden kann. Das erfindungsgemäße Verfahren ermöglicht eine Prognose des Entbindungszeitpunkts ohne Rückgriff auf vorherige Schwangerschaften derselben Frau. Vielmehr wird eine Mustererkennung durch Analyse des zeitlichen Verlaufs von wenigstens einem signifikanten Körperparameter durchgeführt. Wenigstens einer der ausgewerteten Körperparameter wird vorteilhafterweise in zeitlichen Abständen von weniger als sechs Stunden, drei Stunden, einer Stunde, 30 Minuten, 15 Minuten, 5 Minuten, 1 Minute, 30 Sekunden, 15 Sekunden oder sogar weniger als 1 Sekunde erfasst. Vorteilhafterweise wird das Verfahren wenigstens oder nur in den letzten sechs, fünf, vier oder drei Wochen vor dem konventionell prognostizierten Entbindungszeitpunkt durchgeführt. Die Erfindung stellt weiterhin ein computerimplementiertes Verfahren zur Prognose des Entbindungszeitpunktes einer schwangeren Frau bereit, bei dem wenigstens zwei, vorteilhafterweise aber fünf, noch vorteilhafterweise mehr als zehn, und noch vorteilhafterweise mehr als zwanzig Körperparameter der schwangeren Frau zu nur einem Zeitpunkt erfasst werden, und eine Mustererkennung bezüglich Korrelationen der erfassten Körperparameter durch eine Berechnungseinheit durchgeführt werden, wobei ein prognostizierter Entbindungszeitpunkt oder -zeitraum auf Basis der Mustererkennung berechnet wird. Bei den erfassten Körperparametern kann es sich um die vorangehend bereits diskutierten Körperparameter handeln, wobei deren Korrelation auch Aussagen über den zeitlichen Verlauf der Schwangerschaft hinsichtlich des Entbindungszeitpunktes geben kann. So können Korrelationen bezüglich der Vorstufen von Hormonen bzw. bezüglich deren Abbauprodukten Aufschlüsse hinsichtlich des Zeitverlaufs geben.

Es ist möglich, durch Erfassen einer Vielzahl von Körperparametern zusammen mit dem tatsächlichen Entbindungszeitpunkt einen Parametersatz für die Mustererkennung zu optimieren, insbesondere einen Parametersatz für ein künstliches neuronales Netz.

Bei dem wenigstens einen Körperparameter kann es sich insbesondere um eine Stoffkonzentration in einer Körperflüssigkeit der schwangeren Frau handeln, insbesondere im Urin, Blut, Speichel oder Scheidensekret. Insbesondere wird die Stoffkonzentration von wenigstens einem der folgenden Stoffe als Körperparameter erfasst: Corticotropin-releasing Hormon (CRH), adrenocorticotropes Hormon, Kortisol, Progesteron, Östrogen, inflammatorische Zytokine, Prostaglandin, Glukokortikoide, Dehydroepiandrosteron, C-reaktives Protein, Leukozyten und/oder Oxytocin. So ist insbesondere ein Abfall der Progesteron-Konzentration ein deutlicher Hinweis auf eine nahende Niederkunft. Insbesondere verschiebt sich dabei das Progesteron-Östrogen-Verhältnis hin zu einem Östrogen-Übergewicht. Der Progesteron-Abfall kann auch mittelbar über eine andere Stoffkonzentration erfasst werden. So ist beispielsweise alternativ eine Erfassung eines Anstiegs der Östrogen-Konzentration, oder der Östrogen-Vorstufe Dehydroepiandrosteron (DHEA) ein Indikator für einen Abfall der Progesteron-Konzentration. Insbesondere ist es möglich, durch Erfassung von Konzentrationen von verschiedenen Stoffen, die beispielsweise Vorstufen zueinander darstellen, einen zeitlichen Verlauf einer Stoffkonzentration zu erfassen mit nur einer Messung zu einem Zeitpunkt.

Weiterhin kann es sich bei wenigstens einem der Körperparameter um einen Vitalparameter (insbesondere Puls, Durchblutung, Körpertemperatur, Atemfrequenz, elektrischer Widerstand der Haut und/oder Blutdruck) handeln. Eine Erfassung dieser Werte ist vergleichsweise einfach, insbesondere durch ein tragbares Gerät, wie beispielsweise ein Armband, und kann somit in geringen Zeitabständen und automatisiert erfolgen. Insbesondere ist hier der Verlauf der Körpertemperatur von Interesse. So kann es insbesondere zu einer Verringerung der Temperatur an den Tagen vor der Geburt kommen, in Kombination mit einem Temperaturanstieg direkt vor der Geburt. Diese Temperaturverläufe sind im medizinischen und veterinärmedizinischen Bereich für die Prognose des Entbindungszeitpunktes bekannt, allerdings kann es zu ähnlichen Verläufen auch vorher im Verlauf der Schwangerschaft kommen, sodass ohne ärztliche Expertise eine Fehlvorhersage bezüglich einer möglicherweise bevorstehenden Entbindung erfolgen könnte, was zu einer Verunsicherung der betreffenden schwangeren Frau führen könnte. Deswegen muss hier eine exakte und feingliedrige Erfassung der Körpertemperatur erfolgen, und eine entsprechend genaue Mustererkennung, wie sie durch das erfindungsgemäße computerimplementierte Verfahren ermöglicht wird.

In einer bevorzugten Ausführungsform werden als Körperparameter die Körpertemperatur und die Progesteronkonzentration erfasst. In anderen bevorzugten Ausführungsformen werden zusätzlich noch die Konzentrationen von Östrogen, einem Zytokin und/oder des Corticotropin-releasing Hormon (CRH) als Körperparameter erfasst.

In einer Ausführungsform kann das computerimplementierte Verfahren das Erfassen einer manuellen Eingabe umfassen, und zwar eines zusätzlich bestimmten Körperparameters, und das Berechnen des prognostizierten Entbindungszeitpunktes oder -zeitraums erfolgt unter Berücksichtigung des zusätzlich bestimmten Körperparameters. Bei dem zusätzlich bestimmten Körperparameter kann es sich insbesondere um eine Wehenfrequenz und/oder wenigstens einen Parameter aus einer Kardiotokographie handeln. Vorteilhafterweise handelt es sich bei dem zusätzlich bestimmten Körperparameter, um einen Körperparameter, der regelmäßig bei einer Schwangerschaftsvorsorgeuntersuchung erfasst wird, oder zusätzlich erfasst werden kann. Wenn der zusätzlich bestimmte Körperparameter im computerimplementierten Verfahren in Ergänzung zu den zu mehreren Zeitpunkten erfassten Körperparametern zur Verfügung steht, so kann die Berechnung des prognostizierten Entbindungszeitpunktes oder -zeitraums angepasst bzw. genauer gefasst werden. In einer Ausführungsform des Verfahrens erfolgt eine manuelle Eingabe des zusätzlich bestimmten Körperparameters durch medizinisches Personal im Rahmen einer Schwangerschaftsvorsorgeuntersuchung, woraufhin eine automatische Aktualisierung des prognostizierten Entbindungszeitpunktes oder -zeitraums erfolgt.

Vorteilhafterweise werden wenigstens zwei verschiedene Körperparameter erfasst, wobei die Mustererkennung eine Kennung von Korrelationen in den Verläufen dieser wenigstens zwei Körperparameter umfasst. Insbesondere kann dabei wenigstens ein einfach zu erfassender Körperparameter, beispielsweise ein Vitalparameter, in geringen Zeitabständen, beispielsweise wenigstens einmal pro Stunde, erfasst werden, unterstützt durch eine Erfassung von wenigstens einem schwieriger zu erfassenden Körperparameter, beispielweise einer Stoffkonzentration in einer Körperflüssigkeit, in größeren Abständen, beispielsweise wenigstens jeden Tag oder zweiten Tag. Durch diese Kombination wird bei geringfügig erhöhtem Aufwand, die Prognosegenauigkeit deutlich erhöht.

In einer Ausführungsform erfolgt eine Anzeige des prognostizierten Entbindungszeitpunkts oder - zeitraums mindestens drei Tage vor dem prognostizierten Entbindungszeitpunkt bzw. -zeitraum. In weitergehend bevorzugten Ausführungsformen des Verfahrens erfolgt die Anzeige mindestens fünf Tage, mindestens zehn Tage, mindestens zwei Wochen, oder mindestens vier Wochen vor dem prognostizierten Entbindungszeitpunkt. Die Anzeige kann umso früher erfolgen, je besser die Datenbasis für die Mustererkennung ist, also die Anzahl der erfassten Körperparameter, deren Aussagekraft, deren Anzahl pro Zeiteinheit, sowie der Optimierungsgrad des Parametersatzes der Mustererkennung.

In einer Ausführungsform erfolgt die Auswahl, welcher oder welche Körperparameter erfasst werden, in Abhängigkeit von der verbleibenden Zeit bis zum prognostizierten Entbindungszeitpunkt bzw. -zeitraum. So können manche Körperparameter, wie beispielsweise Stoffkonzentrationen in einer Körperflüssigkeit, insbesondere Hormonwerte, eine eher langfristige Prognose ermöglichen, wohingegen andere Körperparameter, beispielsweise Vitalparameter, insbesondere die Körpertemperatur oder Herzfrequenz, eine kurzfristigere Prognose hinsichtlich des Entbindungszeitpunkts oder -zeitraums ermöglichen. Somit ist es dann möglich, die eher langfristig prognostizierenden Körperparameter bei größerer verbleibender Zeit bis zum prognostizierten Entbindungszeitpunkt bzw. -zeitraum vornehmlich oder ausschließlich zu erfassen, und die kürzerfristig prognostizierenden Körperparameter bei kürzerer verbleibender Zeit bis zum prognostizierten Entbindungszeitpunkt bzw. -zeitraum.

In einer Ausführungsform wird der tatsächliche Entbindungszeitpunkt automatisch oder durch manuelle Eingabe erfasst. Ausgehend davon werden eine Vielzahl von Datensätzen von erfassten Körperparametern in ihrem zeitlichen Verlauf hin zum tatsächlichen Entbindungszeitpunkt zum Erkennen von Mustern ausgewertet, die Muster gespeichert und für die Mustererkennung bereitgestellt. Insbesondere wird ein Parametersatz für die Mustererkennung gespeichert und bereitgestellt. Das Erfassen des tatsächlichen Entbindungszeitpunkts kann automatisch, beispielsweise ausgelöst durch erfasste Vitalparameter, die für eine Entbindung charakteristisch sind, erfolgen, oder durch eine manuelle Eingabe durch die Benutzerin oder durch Dritte, insbesondere medizinisches Personal. Bevorzugt erfolgt die erfindungsgemäß durchgeführte Mustererkennung durch künstliche neuronale Netzwerke. Der Parametersatz für die Mustererkennung kann beispielsweise der Parametersatz eines künstlichen neuronalen Netzwerks sein, der durch einen Optimierungsalgorithmus auf Basis der erfassten Körperparameter als Eingangsdaten und der erfassten tatsächlichen Entbindungszeitpunkte als Ausgangsdaten durch Optimierung gewonnen wird. Künstliche neuronale Netzwerke ermöglichen, insbesondere wenn eine Vielzahl von Datensätzen vorliegen, Korrelationen zu ermitteln, die mittels deduktiven Verfahren oder konventioneller Datenauswertung nicht ermittelbar wären, und die Prognosegenauigkeit erheblich verbessern. In einer Ausführungsform wird ein einschichtiges, nicht-rekurrentes künstliches neuronales Netz eingesetzt. In anderen Ausführungsformen können auch mehrschichtige, nicht-rekurrente Netze vorgesehen werden, mit einer oder mehreren verdeckten Schichten von künstlichen Neuronen vor der Ausgabeschicht.

In anderen Ausführungsformen ist es möglich, andere Algorithmen der künstlichen Intelligenz bzw. des maschinellen Lernens einzusetzen.

In einer Ausführungsform kann durch Speichern von spezifischen Parametern bzgl. der schwangeren Frau oder deren Umwelt, eine Gewichtung der Musterauswertung in Abhängigkeit der spezifischen Parameter erfolgen. So kann insbesondere eine Optimierung einer Eingangsschicht eines künstlichen neuronalen Netzwerks hinsichtlich der spezifischen Parameter optimiert werden, und die inneren Schichten hinsichtlich der Körperparameter. Durch eine derartige Optimierung der Topologie des künstlichen neuronalen Netzwerks können erhebliche Verbesserungen bezüglich der Mustererkennung erreicht werden.

Die Erfindung stellt weiterhin ein elektronisches System zur Prognose des Entbindungszeitpunkts einer schwangeren Frau bereit, das wenigstens einen Sensor zum Erfassen wenigstens eines veränderlichen Körperparameters aufweist, und eine Berechnungseinheit, die ausgelegt ist, auf Basis einer Mustererkennung in der zeitlichen Entwicklung des wenigstens einen erfassten Körperparameters einen prognostizierten Entbindungszeitpunkt oder -zeitraum zu bestimmen. Vorteilhafterweise ist die Vorrichtung ausgelegt, erst ab einer vorbestimmten Genauigkeit der Prognose den prognostizierten Entbindungszeitpunkt bzw. -zeitraum anzuzeigen.

Die Erfindung stellt weiterhin ein elektronisches System zur Prognose des Entbindungszeitpunkts einer schwangeren Frau bereit, mit wenigstens einem Sensor zum Erfassen von wenigstens zwei veränderlichen Körperparametern, und einer Berechnungseinheit, die ausgelegt ist, auf Basis einer Mustererkennung der erfassten Körperparameter einen prognostizierten Entbindungszeitpunkt oder -zeitraum zu bestimmen. Vorteilhafterweise werden wenigstens fünf, wenigstens zehn, wenigstens zwanzig oder wenigstens fünfzig Körperparameter als Basis für die Mustererkennung herangezogen.

Vorteilhafterweise umfasst das elektronische System ein tragbares Gerät, insbesondere ein Armband, wobei der wenigstens eine Sensor in dem tragbaren Gerät angeordnet ist, und wobei der wenigstens eine Sensor ein Pulssensor, Durchblutungssensor, Temperatursensor, Hautwiderstandssensor und/oder Beschleunigungssensor ist. Durch die Erfassung der Perfusion mittels des Durchblutungssensors kann die Östrogen- bzw. Progesteron-Konzentration im Blut indirekt bestimmt werden, da diese Hormone die Durchblutung in Abhängigkeit ihrer Konzentration verändern.

Insbesondere umfasst das elektronische System eine Auswerteeinheit, in der der wenigstens eine Sensor in Form eines Biosensors für eine Körperflüssigkeit angeordnet ist. Bei der Auswerteeinheit kann es sich beispielsweise um eine stiftartige Vorrichtung handeln, die in einer abgegebenen Körperflüssigkeit oder in einer Körperöffnung angeordnet werden kann.

Das elektronische System umfasst vorteilhafterweise ein Eingabefeld, über das ein gemessener Körperparameter durch eine manuelle Eingabe erfassbar ist. So kann das Eingabefeld insbesondere auf dem Armband des elektronischen Systems angeordnet sein.

Insbesondere kann das elektronische System ein Smartphone umfassen, alternativ oder zusätzlich auch einen anderen Computer, beispielsweise einen Laptop-Computer oder Desktop-Computer, in dem die Berechnungseinheit und/oder das Eingabefeld vorgesehen ist.

In einer Ausführungsform ist der wenigstens eine Sensor oder die Berechnungseinheit über eine Kommunikationseinheit mit einem Server verbindbar, wobei der wenigstens eine Sensor oder die Berechnungseinheit ausgelegt ist, wenigstens einen Teil der erfassten Körperparameter über die Kommunikationseinheit an den Server zu übermitteln, und von dem Server das Ergebnis der Mustererkennung bezüglich der übermittelten Daten zu erhalten. Der Server kann die Berechnungseinheit umfassen. Der Parametersatz für die Mustererkennung kann mit jedem an den Server übermittelten Datensatz oder zu gegebenen Zeitpunkten auf Basis der neu erhaltenen Datensätze optimiert werden. Weiterhin ist es möglich diesen optimierten Parametersatz für jede Mustererkennung einzusetzen und somit immer bestmöglichste Ergebnisse zu garantieren. Ergänzend oder alternativ kann das elektronische System in einem Offline-Betriebsmodus betrieben werden. Hierfür weist die Berechnungseinheit einen Speicher auf, in dem ein Parametersatz für die Mustererkennung abgelegt ist, wobei die Berechnungseinheit ausgelegt ist, den prognostizierten Entbindungszeitpunkt oder -zeitraum ausgehend von den gemessenen Körperparametern mit dem hinterlegten Parametersatz zu berechnen. Der Parameterdatensatz kann Übertragung von Daten von einem Server aktualisiert werden.

Insbesondere handelt es sich bei dem Parametersatz um einen Parametersatz für ein künstliches neuronales Netzwerk, um hinterlegte Muster bezüglich Körperparametern, oder um Informationen zu Korrelationen zwischen verschiedenen Körperparametern.

Die Erfindung stellt weiterhin ein Client-Server-System zur Prognose des Entbindungszeitpunkts einer schwangeren Frau bereit, wobei der Client durch ein elektronisches System wie vorangehend beschrieben gebildet wird oder ausgebildet ist, ein computerimplementiertes Verfahren wie vorangehend auszuführen, und wobei ein Server vorgesehen ist, der ausgelegt ist, die wenigstens teilweise übermittelten, vom Client erfassten Körperparameter mit auf dem Server hinterlegten Mustern und Korrelationen abzugleichen, und daraus den prognostizierten Entbindungszeitpunkt oder -zeitraum zu berechnen.

In einer bevorzugten Ausführung des computerimplementierten Verfahrens, wird wenigstens zweimal täglich, vorteilhafterweise morgens und abends eine Körpertemperatur der schwangeren Frau erfasst. Insbesondere verläuft die Körpertemperatur einer schwangeren Frau in einem zirkadianen Rhythmus mit einer höheren Körpertemperatur am Morgen und einer geringeren Körpertemperatur am Abend. Kommt es insbesondere zu einer geringeren Temperatur bei einer Morgenmessung wie bei der vorangehenden Abendmessung der Körpertemperatur, so kann dies signifikant für die Mustererkennung und Berechnung des prognostizierten Entbindungszeitpunkts bzw. -zeitraums sein. Eine solche Umstellung der zirkadianen Rhythmik ist insbesondere in einem Bereich ab fünf, ab vier oder ab drei Tage vor einem durch andere Muster prognostizierten Entbindungszeitpunkt signifikant. Weiterhin ist es möglich als Körperparameter über eine Stoffkonzentration in eine Körperflüssigkeit den Grad von zellulären Entzündungsreaktionen zu bestimmen, die ebenfalls aussagekräftig bezüglich der Prognose eines Entbindungszeitpunkts sein können.

Ergänzend kann wenigstens alle drei Tage, wenigstens alle zwei Tage, oder einmal pro Tag ein Progesteron-Wert der schwangeren Frau erfasst werden. Durch eine Mustererkennung bezüglich des Temperatur- und Progesteron-Verlaufs kann dann eine Berechnung des prognostizierten Entbindungszeitpunkts oder -zeitraums erfolgen, vorteilhafterweise wenigstens ab drei Tage vor der Geburt. Ein Absinken der Progesteron-Konzentration führt zu einer Reduktion der Körpertemperatur durch den Wegfall des hyperthermen Effekts des Progesteron. Dies tritt insbesondere in den 48, 36 oder 24 Stunden vor der Geburt auf. Insbesondere ist ein Progesteron-Abfall von 6 ng pro Millimeter auf 2 ng pro Millimeter signifikant. So kann das computerimplementierte Verfahren ausgelegt sein, eine Reduktion der Progesteron-Konzentration auf unter 50 %, unter 40 %, unter 30 % oder unter 20 % ausgehend von einem Ausgangswert als signifikant für die Mustererkennung zu bestimmen. Der Progesteron-Abfall steigert die Wirkung der Östrogene, und führt zu einer Erhöhung der Kontraktilität der glatten Muskulatur am Uterus.

Als ergänzende Information kann auf Basis des letzten Zyklus der Frau gemäß der Naegele-Regel ein theoretischer Geburtstermin berechnet werden, und bei der Berechnung des prognostizierten Entbindungszeitpunkts oder -zeitraums berücksichtigt werden.

Zudem können ergänzend durch Ultraschallbiometrie erfasste Messgrößen bezüglich des ungeborenen Kindes, beispielsweise der Querdurchmesser des Brustkorbes (MAD), der Länge von Scheitel zu Steiß (SSL) oder der biparietalen Durchmessers (BPD) des ungeborenen Kindes, also Körperparameter des ungeborenen Kinds, bei der Mustererkennung berücksichtigt werden.

Insbesondere kann eine Auswerteeinheit vorgesehen sein, die zur Aufnahme von Teststreifen ausgelegt ist, mit denen Stoffkonzentrationen in einer Körperflüssigkeit erfasst werden können. Die Auswerteeinheit kann insbesondere ein Display aufweisen, und kann Messwerte speichern bzw. auswerten, sowie an die Mustererkennung übertragen. Es ist auch möglich, dass es sich bei dem Biosensor um ein Kurzzeitimplantat handelt, das beispielsweise in die Vagina eingeführt wird, und dort die Konzentrationen wenigstens eines Hormons, insbesondere die Progesteron-Konzentration, bevorzugt aber die Konzentrationen von wenigstens 3, 5 oder 10 Hormonen, oder die Körpertemperatur bestimmen kann.

Die Erfindung wird im Folgenden anhand einer beispielshaften Ausführungsform erläutert, mit Bezug auf die folgenden Figuren:
- Fig. 1 zeigt: eine Ausführungsform eines elektronischen Systems gemäß der Erfindung mit einer schwangeren Frau,
- Fig. 2 zeigt: die zeitliche Abfolge der Veränderungen von Körperparametern, die als Grundlage für die Mustererkennung für die Prognose des Entbindungszeitpunkts oder -zeitraums in Ausführungsformen des erfindungsgemäßen Verfahrens bzw. Systems genutzt werden können.

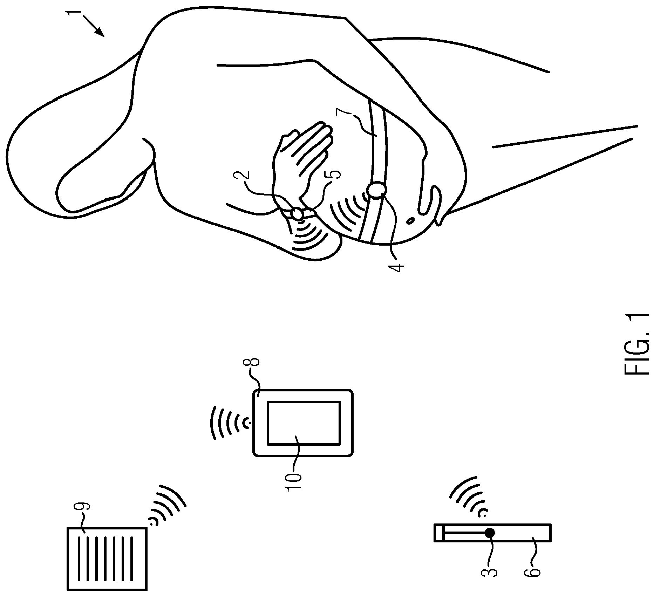

In Figur 1 ist eine schwangere Frau 1 gezeigt, mit einer Ausführungsform eines elektronischen Systems zur Prognose des Entbindungszeitpunkts. Das elektronische System weist mehrere Sensoren 2, 3, 4 auf, mit denen Körperparameter der schwangeren Frau erfasst werden können. Insbesondere handelt es sich hierbei um einen Sensor 2 in einem Armband 5, zur Erfassung von Vitalparametern, insbesondere Puls, Durchblutung, Körpertemperatur, elektrischer Widerstand der Haut und/oder Blutdruck.

Ein weiterer Sensor 3 ist in einer Auswerteeinheit 6 angeordnet. Bei diesem Sensor kann es sich beispielsweise um einen Fotosensor handeln, der in die Auswerteeinheit eingeführte Teststreifen auswertet. Die Teststreifen können insbesondere eine Körperflüssigkeit der schwangeren Frau ausgesetzt worden sein, und bezüglich Stoffkonzentrationen darin verschiedene lokale Farbveränderungen aufweisen, die dann durch den Fotosensor erfasst werden können, sodass eine Stoffkonzentration in der Körperflüssigkeit oder mehrere Stoffkonzentrationen bestimmt werden können. Alternativ kann der Sensor 3 ein Biosensor sein, mit dem direkt Stoffkonzentrationen in einer Körperflüssigkeit der schwangeren Frau ermittelt werden können.

Der weitere Sensor 4 kann in einem Bauchgurt 7 angeordnet sein. Damit können weitere Vitalparameter, wie beispielsweise Atemfrequenz, oder Wehenfrequenz bestimmt werden, oder Körperparameter des ungeborenen Kindes.

Das Armband 5, die Auswerteeinheit 6, und oder der Bauchgurt 7 können jeweils mit einem Funkmodul versehen sein, mit dem die erfassten Körperparameter übertragen werden können. Insbesondere können die erfassten Körperparameter zu einem Smartphone 8 übertragen werden, und dort zusammengeführt werden. Alternativ ist es aber auch möglich, dass die Daten in einem oder allen von Armband 5, Auswerteeinheit 6 oder Bauchgurt 7 empfangen und zusammengefasst werden. Das Smartphone 8, das Armband 5, die Auswerteeinheit 6 und/oder der Bauchgurt 7 weisen eine Berechnungseinheit auf, die ausgelegt ist, auf Basis einer Mustererkennung von einem oder mehr erfassten Körperparameter einen prognostizierten Entbindungszeitpunkt oder - zeitraum zu bestimmen. Alternativ können die bestimmten Körperparameter an einen Server 9 übertragen werden, und die Mustererkennung auf dem Server durchgeführt werden. Weiterhin kann das Smartphone 8, das Armband 5, die Auswerteeinheit 6, und/oder der Bauchgurt 7 eine Anzeigeeinheit 10 aufweisen, auf der der prognostizierte Entbindungszeitpunkt oder-zeitraum angezeigt wird. Alternativ oder zusätzlich kann eine Benachrichtigung, beispielsweise durch ein Tonsignal oder Vibrieren des Smartphones 8, des Bauchgurts 7, der Auswerteeinheit 6, und/oder des Armbands 5 erfolgen.

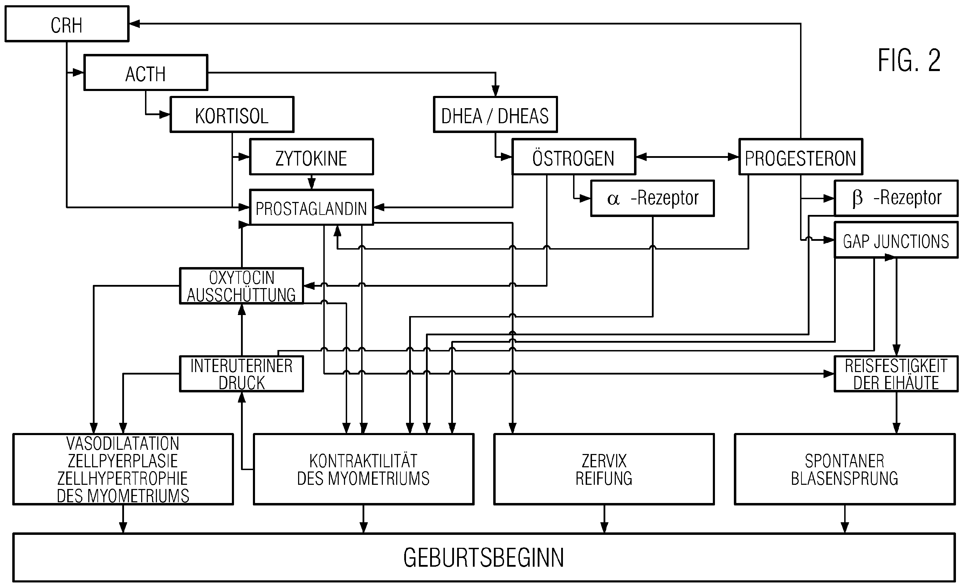

In Fig. 2 ist der kaskadische Ablauf hin zum Geburtsbeginn unter Angabe von relevanten Körperparametern dargestellt. Durch wenigstens teilweise Erfassung dieser Körperparameter, wenigstens in den Abschnitten des Ablaufs in denen diese Körperparameter relevant sind, insbesondere signifikante Veränderungen zeigen, kann eine erfindungsgemäße Prognose des Entbindungszeitpunkts bzw. -zeitraums erfolgen. Während der Schwangerschaft stellt sich ein organisiertes Gleichgewicht (OG) zwischen schwangerschaftserhaltenden und geburtsgeschehenfördernden Körperparameternein. Hier spielen auto-, para- und endokrine Vorgänge eine Rolle (HH-NNR der Mutter bzw. des Fetus und aus dem fetomaternalen Grenzbereich).. Weitere relevante Körperparameter sind der Neurotransmitter CRH, die Steroidhormone Progesteron und Östrogen, die Glukokortikoide, Oxytozin, sowie die Prostaglandine, Zytokine u.a.

Geburtsfördernde Hormone sind insbesondere CRH, ACTH, DHEA, Östrogen, Östradiol, Zytokine IL-1β, II-6, IL-8, Prostagladin PGE₂ PGF_{2α} und Oxytozin.

Geburtshemmende Hormone und Mediatoren sind insbesondere Progesteron und Prostazyklin PGI₂.

Für die Auslösung des Geburtsgeschehens sind die Veränderung der Eihäute (Blasensprung), des Myometriums (Kontraktion) und der Zervix (Reifung) relevant. Im Folgenden sollen die Vorgänge, die zu diesen Veränderungen führen, dargestellt werden.

Der grundlegende Ablauf der Geburtsauslösung folgt einem kaskadischen Ablauf, der. zu einer Verschiebung des organisierten Gleichgewichts (OG) und kann so zur früh- bzw. rechtzeitigen oder über den Termin gehenden Geburt führen. Je näher die Entbindung ist, umso stärker kommt es zu Veränderungen im Gewebe (Dezidua, Myometrium, Zervix), die hauptsächlich Ausdruck einer Inflammation bzw. Infektion mit Veränderungen im mikrobiologischen und im makrobiologischen Bereich sind, und über inflammations- bzw. infektionsindikative Körperparameter bestimmt werden können.

Folgende Körperparameter sind vorteilhafterweise zu berücksichtigen:
I. Ausschüttung von CRH und Reifung des fetalen HH-NNR
II. Inflammation bzw. Infektionen
III. Verschiebung des Progesteron-Östrogen-Verhältnis
IV. Erhöhte Prostaglandin-Synthese
V. Steigerung des intrauterinen Drucks
VI. Oxytocin-Ausschüttung

Im mikrobiologischen Bereich kann es zu qualitativen und quantitativen Veränderungen im Keimspektrum (Bakterien, Viren, Pilze, u.a.) kommen. Desgleichen hat der Stoffwechsel der Keime z.B. die Protease Bildung - mit Auswirkung auf den Arachidon- und Zitronensäurezyklus - einen erheblichen Einfluss. Im makrobiologischen Bereich - also im Menschen - lösen die in enger Verbindung mit dem mikrobiologischen Bereich stattfindenden Vorgänge eine immunologische Kaskade mit zellgebundenen und humoralen Antworten (Zytokine, etc.) aus. Dies kann über geeignete Körperparameter bestimmt werden. Dazu im Detail:

### I. Ausschüttung von CRH und Reifung des fetalen HH-NNR

Während der Schwangerschaft wird in Geweben des fetomaternalen Grenzbereichs, der Plazenta, des Chorions, den Zellen des Amnions und der Dezidua das corticotropin-releasing hormon (CRH) produziert. Dies führt beim Fetus zur Reifung des fetalen HH-NNR und zur Produktion des adrenocorticotropen Hormons (ACTH) im fetalen Hypophysenvorderlappen. Dies führt wiederum zu einer vermehrten Ausschüttung von Kortisol, dessen Konzentration als Körperparameter bestimmt werden kann. Weiterhin aktiviert der erhöhte Kortisol-Spiegel Entzündungszellen und setzt damit vermehrt inflammatorische Zytokine und Prostaglandine frei, deren Konzentrationen als Körperparameter bestimmt werden können. Das CRH hat zudem eine Prostaglandin-verstärkende Wirkung und führt, bei höheren Spiegeln, zu einer Vasodilatation und Kontraktilitäts-Erhöhung des Myometriums.

### II. Inflammation bzw. Infektion

Der gemäß vorgenannten Mechanismen oder als Seiteneinstieg durch eine Infektion im Körper (z.B. Parodontose oder des Amnions durch pathogene bzw. fakultativ pathogene Keime) erhöhte Kortisol-Spiegel führt bei Infektionen lokal zu Entzündungen und allgemein zu einer Ausschüttung von Zytokinen wie -1,-6,-8,-10,-12, u.a. oder Tumornekrosefaktoren sowie Granulozytenkolonienstimulierende Faktoren. Eine Erhöhung der Leukozytenzahl ist eine der Folgen. Zusammen mit der entzündungsinduzierten Stimulation des Arachidonsäure-Metabolismus wird die Prostaglandinproduktion erhöht, was im Rahmen der Körperparametererfassung bestimmt werden kann. Dies führt zur Zervixreifung und (vorzeitigen) Wehen. Die Entzündung am Eipol im Rahmen der Amnion-Infektion führt auch zur Schädigung der Eihäute und erhöht die Gefahr des Blasensprungs.

### III. Verschiebung des Progesteron-Östrogen-Verhältnis

Die Produktion von ACTH erhöht die Ausschüttung der Östrogenvorstufe Dehydroepiandrosteron (DHEA). Auch Glukokortikoiden können indikativ für den Geburtsbeginn sein. Die DHEAS steigt parallel zum Östrogen zum Ende der Schwangerschaft. Dadurch verschiebt sich das Progesteron-Östrogen-Verhältnis hin zu einem Östrogenübergewicht. Dies hat zwei Auswirkungen. Östrogen wirkt kontraktrationsfördernd auf die α-Rezeptoren und hat einen positiven Einfluss auf die Prostaglandin- und Oxytocin-Rezeptoren im Myometrium. Progesteron hingegen aktiviert die β-Rezeptoren, was eine relaxierende Wirkung entfacht. Progesteron hat eine unterdrückende Wirkung auf die Synthese der gapjunctions sowie auf die CRH-Produktion. Gegen Ende der Schwangerschaft kommt es zudem zu einem Rückgang der Progesteron-Produktion der Plazenta was wiederum ein weiterer geburtsfördernder Baustein ist und auch Auswirkung auf die Thermogenese hat.

### IV. Erhöhte Prostaglandin-Synthese

Progesteron wird in der Plazenta zu Östrogen umgewandelt und stimuliert die Produktion von Prostaglandin F₂α (PGF₂α) im Myometrium. Die Prostaglandine induzieren im Myometrium Kontraktionen und erhöhen damit den intrauterinen Druck und bewirken eine Vasodilatation des Myometriums. So beeinflussen sie auch die Zervixreifung und den spontanen Blasensprung.

### V. Steigerung des intrauterinen Drucks

Der intrauterine Dehnungsreiz führt zur Zellhyperplasie und Zellhypertrophie im Myometrium und zur Expressionssteigerung von kontraktionsassoziierten Proteine (GAP-Junctions Connexin H3) sowie zur Beeinträchtigung der Reißfestigkeit der Eihäute.

### VI. Oxytocin-Ausschüttung

Die Weitung des Muttermundes führt zur Oxytocin-Ausschüttung des maternalen Hypophysenhinterlappens. Das Oxytocin wiederum stimuliert die Prostaglandin-Synthese und induziert zusätzliche Kontraktionen des Myometriums um den Fetus vollständig aus dem Uterus auszutreiben. Inbesondere können die folgenden Stoffe bzw. deren Konzentrationen als Körperparameter erfasst werden: CRH, ACTH, Kortisol, Zytokine, Prostaglandin, DHEA/ DHEAS, Ötrogen, α -Rezeptor, Progesteron, β -Rezeptor und/oder Oxytocin. Mit Beginn der Geburt weisen die Muskelzellen des Uterus Gap Junctions auf, wodurch diese zu einem Synzytium verbunden werden, und sich synchron kontrahieren können.

Durch die in Fig. 2 gezeigten Abläufe kommt es zu einer Vasodilatation, Zellhyperplasie, Zellhypertrophie des Myometriums, zu Kontraktilität des Myometriums, zur Zervixreifung und/oder zu einem spontanen Blasensprung. Diese Ereignisse sind wiederum direkte Vorstufen des Geburtsbeginns.

Zudem gibt es Seiteneinstiege in der Geburtskaskade, die ebenfalls durch die Bestimmung von Körperparametern erfasst werden können:

### A. Stress oder hypoxisch-ischämische Läsionen der Plazenta

Chronischer mütterlicher Stress kann die fetale HH-NNR-Achse vorzeitig aktivieren indem die CRH Freisetzung aktiviert wird und Zytokine ausgeschüttet werden. Mit zunehmender Schwangerschaftsdauer kann es zu einer Desensibilisierung gegenüber einem erhöhten CRH-Spiegel und damit zum Abbau des Stresstraumas kommen.

### B. Auswirkungen des zirkadinen Rhythmus auf Interleukin IL-1β und TNF

Es kommt zu einer erhöhten Aktivität des Myometriums in der zweiten Nachthälfte und am frühen Morgen, sowie zu erhöhten Progesteron- und DHEAS-Spiegeln, sowie maternalen Estradiol- und Progesteronschwankungen.

Die vorgenannten Veränderungen im Körper der schwangeren Frau spiegeln sich in ihren Körperparametern wieder, sei es direkt, beispielsweise in der Stoffkonzentration eines Hormons, oder indirekt, beispielsweise in der Veränderung eines Vitalparameters. So kann eine Entzündungsreaktion zu erhöhter Körpertemperatur und/oder veränderten Pulswerten führen. Die Erfassung von wenigstens einem Körperparameter über die Zeit und/oder von mehreren verschiedenen Körperparametern und eine diesbezügliche Mustererkennung, insbesondere hinsichtlich Korrelationen zwischen den Körperparametern, kann somit eine genaue Prognose des Entbindungszeitpunkts bzw. -zeitraums ermöglichen.

## Patentansprüche

1. Computerimplementiertes Verfahren zur Prognose des Entbindungszeitpunkts einer schwangeren Frau, mit den Schritten:
Erfassen von wenigstens einem Körperparameter der schwangeren Frau (1) zu mehreren Zeitpunkten,
Durchführen einer Mustererkennung bezüglich des zeitlichen Verlaufs des wenigstens einen Körperparameters durch eine Berechnungseinheit, und
Berechnen eines prognostizierten Entbindungszeitpunkts oder -zeitraums auf Basis der Mustererkennung.

2. Computerimplementiertes Verfahren gemäß Anspruch 1, wobei es sich bei wenigstens einem der Körperparameter um eine Stoffkonzentration in einer Körperflüssigkeit der schwangeren Frau handelt, insbesondere von wenigstens einem der folgenden Stoffe: Corticotropin-releasing Hormon, adrenocorticotropes Hormon, Kortisol, Progesteron, Östrogen, inflammatorische Zytokine, Prostaglandin, Glukokortikoide, Dehydroepiandrosteron, C-reaktives Protein, Leukozyten und/oder Oxytocin.

3. Computerimplementiertes Verfahren gemäß Anspruch 1 oder 2, wobei es sich bei wenigstens einem der Körperparameter um einen Vitalparameter, insbesondere Puls, Durchblutung, Körpertemperatur, Atemfrequenz, elektrischer Widerstand der Haut und/oder Blutdruck der schwangeren Frau (1) handelt.

4. Computerimplementiertes Verfahren gemäß einem der vorangehenden Ansprüche, umfassend ein Erfassen einer manuellen Eingabe eines zusätzlich bestimmten Körperparameters, insbesondere einer Wehenfrequenz und/oder wenigstens einem Parameter aus einer Kardiotokografie, und Berechnen eines prognostizierten Entbindungszeitpunkts oder -zeitraums unter Berücksichtigung des zusätzlichen Körperparameters.

5. Computerimplementiertes Verfahren gemäß einem der vorangehenden Ansprüche, wobei mehrere Körperparameter erfasst werden, und wobei die Mustererkennung eine Erkennung von Korrelationen in den Verläufen von wenigstens zwei Körperparametern umfasst.

6. Computerimplementiertes Verfahren gemäß einem der vorangehenden Ansprüche, wobei mindestens drei Tage vor dem prognostizierten Entbindungszeitpunkt eine Anzeige des prognostizierten Entbindungszeitpunkts oder -zeitraums erfolgt.

7. Computerimplementiertes Verfahren gemäß einem der vorangehenden Ansprüche, wobei die Auswahl welcher oder welche Körperparameter erfasst werden, in Abhängigkeit von der verbleibenden Zeit bis zum prognostizierten Entbindungszeitpunkt bzw. -zeitraum erfolgt.

8. Computerimplementiertes Verfahren gemäß einem der vorangehenden Ansprüche, **gekennzeichnet durch** Erfassen des tatsächlichen Entbindungszeitpunkts automatisch oder durch manuelle Eingabe, und Auswerten einer Vielzahl von Datensätzen von erfassten Körperparametern in ihrem zeitlichen Verlauf hin zum tatsächlichen Entbindungszeitpunkt zum Erkennen von Mustern, Speichern der Muster, und Bereitstellen der Muster für die Mustererkennung.

9. Computerimplementiertes Verfahren gemäß einem der vorangehenden Ansprüche, **gekennzeichnet durch** Speichern von spezifischen Parametern bezüglich der schwangeren Frau oder deren Umwelt, Gewichtung der Musterauswertung in Abhängigkeit der spezifischen Parameter.

10. Elektronisches System zur Prognose des Entbindungszeitpunkts einer schwangeren Frau (1), **gekennzeichnet durch**
wenigstens einen Sensor (2, 3, 4) zum Erfassen wenigstens eines veränderlichen Körperparameters, und
eine Berechnungseinheit, die ausgelegt ist, auf Basis einer Mustererkennung in der zeitlichen Entwicklung des wenigstens einen erfassten Körperparameters einen prognostizierten Entbindungszeitpunkt oder -zeitraum zu bestimmen.

11. Elektronisches System gemäß Anspruch 10, umfassend ein tragbares Gerät (4, 5, 6), insbesondere ein Armband (5), wobei der wenigstens eine Sensor (2, 3, 4) in dem tragbaren Gerät (4, 5, 6) angeordnet ist, und wobei der wenigstens eine Sensor (2, 3, 4) ein Pulssensor, Durchblutungssensor, Temperatursensor, Hautwiderstandssensor und/oder Beschleunigungssensor ist.

12. Elektronisches System gemäß Anspruch 10 oder 11, umfassend eine Auswerteeinheit (6), in der der wenigstens eine Sensor (3) in Form eines Biosensors für eine Körperflüssigkeit angeordnet ist.

13. Elektronisches System gemäß einem der Ansprüche 10 bis 12, umfassend ein Eingabefeld (10) über das ein gemessener Körperparameter durch eine manuelle Eingabe erfassbar ist.

14. Elektronisches System gemäß einem der Ansprüche 10 bis 13, wobei der wenigstens eine Sensor (2, 3, 4) über eine Kommunikationseinheit mit einem Server (9) verbindbar ist, die ausgelegt ist, wenigstens einen Teil der erfassten Körperparameter über die Kommunikationseinheit an den Server (9) zu übermitteln, und von dem Server (9) das Ergebnis der Mustererkennung bezüglich der übermittelten Daten zu erhalten.

15. Client-Server-System zur Prognose des Entbindungszeitpunkts einer schwangeren Frau, wobei der Client durch ein elektronisches System gemäß einem der vorangehenden Ansprüche 10 bis 14 gebildet wird, und wobei ein Server (9) vorgesehen ist, der ausgelegt ist, die wenigstens teilweise übermittelten, vom Client erfassten Körperparameter mit auf dem Server (9) hinterlegten Mustern und Korrelationen abzugleichen, und daraus den prognostizierten Entbindungszeitpunkt oder -zeitraum zu berechnen.
